# EUROPÄISCHE PATENTANMELDUNG

(11) **EP 3 117 805 A1**
(43) Veröffentlichungstag der Anmeldung: **18.01.2017**
(21) Anmeldenummer: 16178998.7
(22) Anmeldetag: 12.07.2016
(51) Int. Cl.: A61F 5/56

(54) **VORRICHTUNG ZUR VERHINDERUNG VON SCHNARCHEN**

(30) Priorität: 13.07.2015 DE 202015103669 U
(71) Anmelder: Nachtwaechter Schlafprodukte GmbH & Co. KG, 88499 Riedlingen (DE)
(72) Erfinder: Ruoff, Marcus, 88499 Riedlingen (DE)
(74) Vertreter: Baur & Weber Patentanwälte PartG mbB

(57) **Zusammenfassung**

Es wird eine Vorrichtung zur Verhinderung von Schnarchen beschrieben, die einen Grundkörper (GK) aufweist, der um eine Rückenseite (RS) und eine Vorderseite (VS) eines Trägers (TR) anbringbar ist, wobei der Grund-körper (GK) auf der Rückenseite (RS) einen Rückenkörper (RK) aufweist, in den ein vorstehender Hemmkörper (HK) mit im wesentlichen länglicher Form entlang einer Längsachse (LA) eingebracht ist, wobei der Rückenkörper (RK) so angeordnet ist, dass die Längsachse (LA) im wesentlichen senkrecht zu einer Längsachse einer Wirbelsäule (WS) des Trägers (TR) liegt.

## Beschreibung

Die Erfindung betrifft eine Vorrichtung zur Verhinderung von Schnarchen.

Bekanntlich ist Schnarchen häufig mit einer Rückenlage der schlafenden Person verbunden. Neben der Störung der Nachtruhe für andere sich in der Nähe der schnarchenden Person befindenden Schlafsuchenden, führt Schnarchen über sogenannte Schlafapnoe-Zustände auch zu gesundheitlichen Beeinträchtigungen.

Aus dem Stand der Technik sind diverse Ansätze bekannt, die Schnarchen verhindern sollen. Neben Vorrichtungen, die in Verbindung mit der Zunge oder dem Gebiss des Trägers stehen, sind zahlreiche Bekleidungsstücke bekannt, die einen Einfluss auf das Auftreten von Schnarchen haben.

So ist aus der DE 20 2014 003 665 U ein Rucksack zur Verhinderung der Einnahme der Rückenlage im Schlaf bei lageabhängigem obstruktivem Schnarchen beschrieben. Der Rucksack weist ein Rückenteil auf, wobei eine mittig auf das Rückenteil aufgesetzte, verschließbare Tasche zur Aufnahme eines oder mehrerer formstabiler, die Tasche im Wesentlichen ausfüllender Formkörper vorgesehen ist. An Elastikansätzen befestigte Bauchgurte können mit Hilfe eines Klettbandes miteinander verbunden werden. An dem Bauchgurt, welcher das im getragenen Zustand außen befindliche Klettband aufweist, ist zusätzlich eine Abdecklasche mit ein oder mehreren Klettbändern als Abdeckung für das gegebenenfalls frei liegende Klettband dieses Bauchgurtes angebracht.

Die DE 20 2014 003 664 U ist ähnlich zu der oben beschriebenen, wobei hier als Hauptunterscheid anstelle eines Rucksack ein Bauchgurt zur Verhinderung der Einnahme der Rückenlage im Schlaf bei lageabhängigem obstruktivem Schnarchen beschrieben wird.

In der US 8,015,975 B2 wird ein Bekleidungsgegenstand gezeigt, der Schlafen auf dem Rücken zu verhindern sucht. Das Bekleidungsstück kann sowohl als Hemd, als Weste als auch als Rucksack ausgeführt sein. Auf dem Rücken befindet sich eine Tasche, die mit einem Einsatz bestückt wird. Der Einsatz wird zwischen den Schulterblättern positioniert und weist eine entlang der Wirbelsäule angeordnete Zylinderachse auf. Demnach wird ein Träger beim Versuch einer Schlaflage auf dem Rücken in eine Seitenlage zurück gebracht bzw. mittels des Einsatzes in einer Position gehalten, die eine gerade Kopfhaltung ermöglicht.

In der DE 297 03 897 U1 wird eine westenförmige Vorrichtung beschrieben, die auf dem Rücken mit mehreren Stückkörpern versehen ist, die lösbar mit dem Rückenteil verbunden sind. Die Weste wird auf der Vorderseite mittels Klettverschlüsse geschlossen und ist so ausgeführt, dass diese im Wesentlichen unverrückbar anliegt. Neben einer Ausführungsform mit einer punktförmigen Einwirkung auf den Rücken des Trägers ist es auch vorgesehen, die Weste mit einem stegförmigen Körper im Rückenteil auszubilden. Der Körper am Rücken ist wiederum parallel zur Wirbelsäule angeordnet.

Die DE 296 18 276 U1 zeigt einen Brustgürtel für Schnarcher, wobei auch hier verhindert werden soll, dass sich schnarchende Personen im Schlafzustand selbständig in Rückenlage bewegen können. Auf der Rückseite des Brustgürtels befindet sich eine Hülle, die einen Durchmesser von ca. 12 cm aufweist, so dass diese einen festen Gegenstand aufnehmen kann, der für die betreffende Person ein Hindernis in der Rückenlage bedeutet.

In der DE 198 37 096 A1 ist eine Vorrichtung zur Beeinflussung der Position des Körpers eines Menschen gezeigt, bei der diejenige Auflagefläche, deren Lage es zu verhindern gilt, mit einem oder mehreren Elementen versehen wird, die länglich oder in Form eines Tennisballs ausgebildet sind. Die beschriebenen Elemente sollen beim Einnehmen insbesondere einer Rückenlage eine Gegenkraft auf den Körper ausüben. Hierbei ist es insbesondere vorgesehen, die Elemente entlang der Wirbelsäule anzuordnen.

In der DE 196 948 A ist eine Vorrichtung zur Verhütung des Liegens auf dem Rücken gezeigt, die sowohl das sich in dieser Lage oftmals eintretende Schnarchen als auch das Auftreten von erotischen Traumbildern zu verhindern sucht. Dazu wird ein Gürtel bereitgestellt, der zwei gegeneinander federnde Rückenplatten aufweist, so dass bei Rückenlage des Körpers kegelförmige Stifte durch die dem Schlafenden zugewandte Platte durchtreten, so dass dieser geweckt wird und eine andere Körperlage einnimmt.

Bei Verwendung von bekannten Vorrichtungen hat es sich jedoch gezeigt, dass diese die Neigung zu Schnarchen zwar reduzieren, jedoch nicht vollständig verhindern können.

Es ist daher Aufgabe der Erfindung, eine Vorrichtung zur Verhinderung von Schnarchen zu schaffen, die die Neigung zu Schnarchen noch weiter verringert.

Diese Aufgabe wird durch die Merkmale des unabhängigen Patentanspruchs 1 gelöst. Weitere vorteilhafte Ausgestaltungen der Erfindung sind jeweils Gegenstand der Unteransprüche. Diese können in technologisch sinnvoller Weise miteinander kombiniert werden. Die Beschreibung, insbesondere im Zusammenhang mit der Zeichnung, charakterisiert und spezifiziert die Erfindung zusätzlich.

Gemäß der Erfindung wird eine Vorrichtung zur Verhinderung von Schnarchen geschaffen, die einen Grundkörper aufweist, der um eine Rückenseite und eine Vorderseite eines Trägers anbringbar ist, wobei der Grundkörper auf der Rückenseite einen Rückenkörper aufweist, in den ein vorstehender Hemmkörper mit im wesentlichen länglicher Form entlang einer Längsachse eingebracht ist, wobei der Rückenkörper so angeordnet ist, dass die Längsachse im wesentlichen senkrecht zu einer Längsachse einer Wirbelsäule des Trägers liegt.

Im Vergleich zu den bisher bekannten Vorrichtungen wird erfindungsgemäß am Rücken des Trägers ein Hemmkörper so angeordnet, dass dieser im Wesentlichen senkrecht zur Längsachse einer Wirbelsäule am Rücken des Trägers anliegt. Demnach wird über den Hemmkörper die Schlafposition in Rückenlage nicht nur erschwert oder unangenehm gestaltet, sondern gänzlich verhindert. Wie Versuche gezeigt haben, ist eine derartige Vorgehensweise den bisher bekannten Vorrichtungen überlegen. Zwar ist das Vorhandensein des Hemmkörpers mit einer gewissen Eingewöhnung verbunden, sobald diese jedoch überwunden ist, wird zuverlässig die Schlafposition in Rückenlage verhindert, so dass die Neigung zu Schnarchen während des Schlafes weiter reduziert wird. Der Grundkörper ist dabei um eine Rückenseite und eine Vorderseite des Trägers angebracht, wobei der Grundkörper so ausgestaltet ist, dass ein fester Sitz der Vorrichtung erreicht wird. So kann der Grundkörper in seinem Sitz so gewählt werden, dass zwar für einen Träger kein beengtes Gefühl entsteht, aber dennoch ein zuverlässiger Sitz des Rückenkörpers bzw. des Hemmkörpers erreicht wird.

Gemäß einer Ausführungsform der Erfindung weist der Rückenkörper entlang der Längsachse eine Länge auf, die wenigstens ein Drittel der Breite der Rückenseite beträgt.

Demnach kann auch schon bei kleineren Rückenkörpern, die folglich auch nur einen kleineren Hemmkörper aufnehmen können, eine Reduzierung der Schnarchneigung erreicht werden.

Gemäß einer weiteren Ausführungsform der Erfindung, ist die Länge des Rückenkörpers entlang der Längsachse im Wesentlichen gleich zur Breite der Rückenseite gewählt.

Demnach ragt der im Rückenkörper angeordnete Hemmkörper nicht über die seitliche Begrenzung des Rückens des Trägers hinaus, was zur Verhinderung der Rückenlage ausreicht. Die Länge des Rückenkörpers entlang der Längsachse kann auf die Schlafgewohnheiten des Trägers beziehungsweise die Körperform oder Körpergröße des Trägers angepasst sein. In anderen Ausführungsformen der Erfindung kann es vorgesehen sein, dass der Rückenkörper über die Rückenseite hervorsteht. Die gewählte Größe des Rückenkörpers ermöglicht eine zuverlässige Verhinderung der Rückenlage des Trägers.

Gemäß einer weiteren Ausführungsform der Erfindung wird der Rückenkörper entlang der Längsachse mit einem rechteckigen Querschnitt ausgeführt.

Demnach entsteht an den Rändern des Rückenkörpers ebenfalls ein quadratischer oder eine rechteckiger Querschnitt, so dass eine zuverlässige Hemmung gegen ein unbeabsichtigtes oder unkontrolliertes Wechseln in die Rückenlage entsteht.

Gemäß einer weiteren Ausführungsform der Erfindung ist der Rückenkörper mit einem Verschluss versehen, so dass ein auswechselbares Polster in dem Rückenkörper als Hemmkörper angeordnet werden kann.

Die Möglichkeit, den Hemmkörper aus dem Rückenkörper zu entfernen, weist insbesondere Vorteile bezüglich der Hygiene der Vorrichtung auf, da der Grundkörper häufig einer Reinigung unterzogen werden muss, da er mit dem Träger in Verbindung steht. Das Material des Grundkörpers und des Rückenkörpers kann dabei so gewählt werden, dass keine Einschränkungen bezüglich Waschbarkeit bestehen. Durch vorheriges Entfernen des Hemmkörpers ist es daher möglich, dass dieser durch den Waschvorgang nicht beeinflusst wird, so dass beispielsweise Materialeigenschaften wie Kompressibilität, Härte oder dergleichen, nicht beeinflußt werden.

Gemäß einer weiteren Ausführungsform der Erfindung ist der Grundkörper als Weste ausgeführt.

Unter dem Begriff "Weste" wird sowohl ein Kleidungsstück verstanden, das über den Kopf des Trägers gezogen werden muss, als auch ein Kleidungsstück, das beispielsweise über eine frontseitig angeordnete Verschlussmöglichkeit geöffnet und geschlossen werden kann. Eine derartige Vorgehensweise ermöglicht ein komfortables Anlegen und Tragen der erfindungsgemäßen Vorrichtung. Dabei kann die Weste an der Vorderseite mit einem Klettverschluss oder mit einem Reißverschluss versehen sein.

Gemäß einer weiteren Ausführungsform der Erfindung ist der Grundkörper als Bauchgurt oder als Brustgurt ausgeführt.

Auch die Ausführungsform der erfindungsgemäßen Vorrichtung in Form eines Gurtes kann mit geeigneten Verschlussmöglichkeiten versehen sein. Je nach Beschaffenheit des Trägers kann dabei zwischen Brust bzw. Bauchgurt gewählt werden. So kann in manchen Fällen, beispielsweise bei stark übergewichtigen Personen, ein Bauchgurt unangenehm zu tragen sein. In diesem Falle kann auf den Brustgurt ausgewichen werden. Außerdem hat es sich gezeigt, dass bei weiblichen Trägern oftmals die Verwendung eines Bauchgurts angenehmer ist.

Gemäß einer weiteren Ausführungsform der Erfindung ist der Hemmkörper in Richtung der Rückenseite des Trägers profiliert ausgeführt.

Demnach kann die hautnahe Oberfläche des Trägers im Bereich des Hemmkörpers atmungsaktiv gestaltet werden, was insbesondere Schwitzen während des Schlafens stark reduziert.

Gemäß einer weiteren Ausführungsform der Erfindung ist der Hemmkörper als Schaumstoffblock in den Rückenkörper eingebracht.

Ein entsprechend gehärteter Schaumstoff stellt eine kostengünstige und einfache Realisierung des Hemmkörpers dar. Der Grundkörper bzw. der Rückenkörper können aus einem textilen Material hergestellt sein.

Gemäß einer weiteren Ausführungsform der Erfindung ist der Hemmkörper als aufblasbarer oder fixierter Hohlkörper in den Rückenkörper eingebracht.

Ein Hohlkörper stellt ebenfalls eine kostengünstige und einfache Realisierung des Hemmkörpers dar, die insbesondere im Falle eines aufblasbaren Hohlkörpers auch eine gewisse Anpassbarkeit an die Körperform ermöglicht und über die mittels des Aufblasens erzeugte Härte eine weitere Variationsmöglichkeit bietet.

Nachfolgend werden einige Ausführungsbeispiele anhand der Zeichnung näher erläutert. Es zeigen:
- Fig. 1: eine Rückenansicht eines Trägers mit der erfindungsgemäßen Vorrichtung in einer schematischen Darstellung,
- Fig. 2: der Hemmkörper der Vorrichtung aus Fig. 1 in einer Draufsicht,
- Fig. 3A: eine Ausführung der erfindungsgemäßen Vorrichtung als Weste in einer Vorderansicht,
- Fig. 3B: die Ausführungsform gemäß Fig. 3A in Rückansicht,
- Fig. 4: eine erste Ausführungsform eines Brust- oder Bauchgurts in einer Draufsicht, und
- Fig. 5: eine zweite Ausführungsform eines Brust- oder Bauchgurts in einer Draufsicht.

In den Figuren sind gleiche oder funktional gleich wirkende Bauteile mit den gleichen Bezugszeichen versehen.

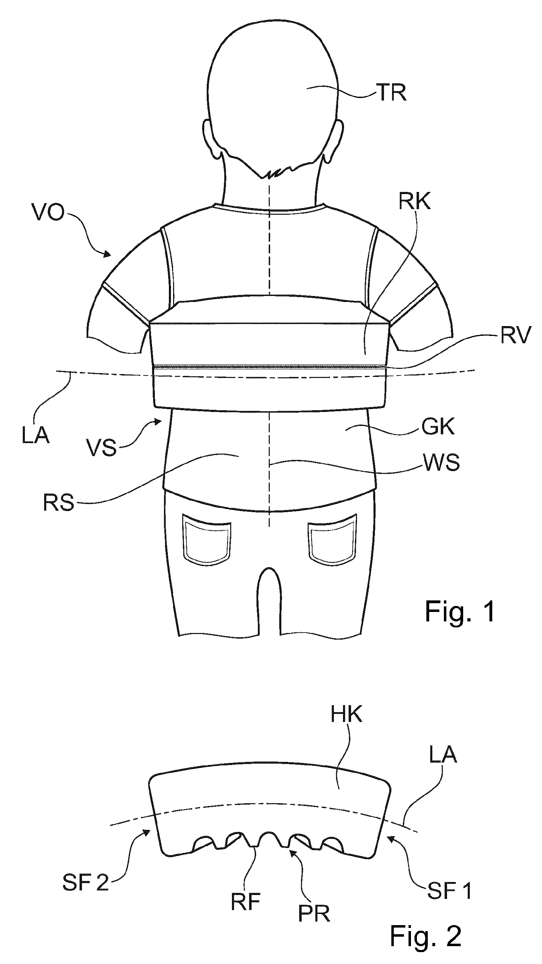

In Fig. 1 ist eine erfindungsgemäße Vorrichtung VO gezeigt, nachdem diese von einem Träger TR angelegt wurde. In Fig. 1 ist die Darstellung dabei so gewählt, dass eine Draufsicht auf eine Rückenseite RS des Trägers TR dargestellt ist. Man erkennt, dass die Vorrichtung VO einen Grundkörper GK aufweist, der die Rückenseite RS des Trägers TR bedeckt. Über entsprechende Öffnungen können sowohl der Kopf als auch die Arme des Trägers TR aus dem Grundkörper GK heraus geführt werden. Die Lage einer Wirbelsäule WS des Trägers TR ist in Fig. 1 mit der vertikalen gestrichelten Linie angedeutet. Auf der Rückenseite RS ist auf dem Grundkörper GK ein Rückenkörper RK angeordnet, der über eine Verschlussvorrichtung RV, die beispielsweise als Reißverschluss, Klettverschluss oder dergleichen gebildet sein kann, mit einem Hemmkörper gefüllt ist. Ausführungsbeispiele für den Hemmkörper werden weiter unten gezeigt.

Erfindungsgemäß ist es vorgesehen, die Längsachse LA im Rückenkörper RK so zu wählen, dass diese im Wesentlichen senkrecht zur Wirbelsäule WS zu liegen kommt. Demnach wird die oftmals mit Schnarchen verbundene Rückenlage beim Träger TR stark reduziert.

Unter Bezugnahme auf Fig. 2 ist ein Ausführungsbeispiel für den Hemmkörper HK gezeigt. Der Hemmkörper HK kann entlang der Längsachse LA einen abschnittsweise rechteckigen Querschnitt aufweisen, so dass die beiden Seitenflächen SF1 und SF2 als Hemmung wirken, so dass der Träger TR nicht in Rückenlage wechseln kann. Die dem Rücken des Trägers TR zugeordnete Rückenfläche RF ist mit einer Profilierung PR versehen, die beispielsweise als Rillen ausgeführt sein kann. Dadurch wird Schwitzen während des Tragens der erfindungsgemäßen Vorrichtung VO durch den Träger TR deutlich reduziert, da über die Profilierung PR Luftzirkulation zwischen dem Hemmkörper HK und dem Rücken des Trägers TR möglich ist.

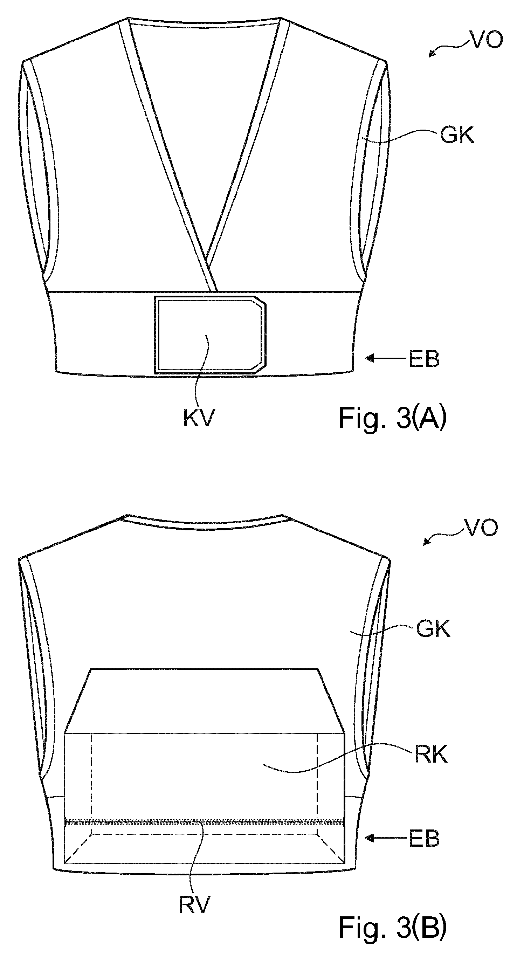

In Fig. 3A und 3B ist eine Vorder- bzw. Rückansicht der erfindungsgemäßen Vorrichtung VO gezeigt. Dieses Ausführungsbeispiel stellt einen Grundkörper GK in Form einer Weste bereit. Auf der Vorderseite kann die Vorrichtung VO einen Verschluss aufweisen, der das einfache Anlegen der Weste ermöglicht. In Fig. 3A ist dieser Verschluss als Klettverschluss KV gezeigt. Im Bereich außerhalb des Klettverschlusses kann ein zumindest abschnittsweise ausgebildeter Elastikbund EB vorgesehen sein, der ein straffes Anlegen des Grundkörpers über den Träger TR unterstützt. Hierbei ist vorzugsweise die Hakenseite des Klettverschluss KV innenliegend, d.h. zum Träger hin gerichtet, so dass keine Abdeckung notwendig ist, um vor versehentlichem Kletten zu schützen. Anstelle des Klettverschlusses KV kann auch ein Reißverschluss, Knöpfe, Haken oder dergleichen vorgesehen sein.

Die in Fig. 3B gezeigte Rückansicht verdeutlicht wiederum die Hemmung der Rückenlage beim Träger TR aufgrund des Rückenkörpers RK, der mit dem Hemmkörper gemäß Fig. 2 gefüllt ist.

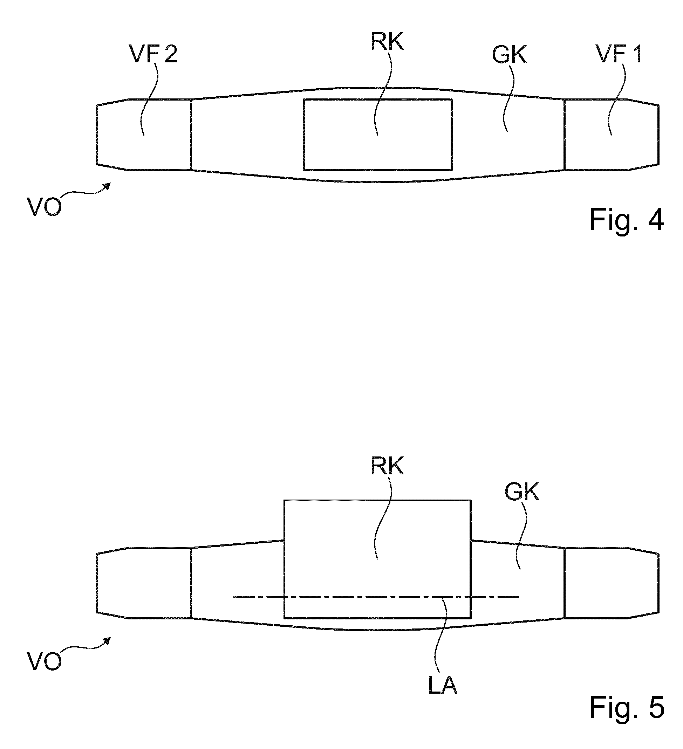

In Fig. 4 ist eine weitere Ausführungsform der erfindungsgemäßen Vorrichtung VO beschrieben. Die Ausführung gemäß Fig. 4 zeigt einen als Elastikbund geformten Grundkörper GK, der als Brustgurt oder als Bauchgurt ausgebildet ist. Der Rückenkörper RK ist dabei zentral am Grundkörper GK angeordnet, wobei die Enden des als Gurt ausgebildeten Grundkörpers GK in eine erste und eine zweite Verschlussfläche VF1 und VF2 münden, die beispielsweise mit entsprechenden Klettverschlüssen versehen sein können. Die Verwendung anderer Verschlussmöglichkeiten ist selbstverständlich ebenfalls möglich. Der Rückenkörper kann wiederum mit einer Verschlussvorrichtung versehen sein, um den darin enthaltenen Hemmkörper HK austauschbar anordnen zu können.

In Fig. 5 ist eine zur Fig. 4 ähnliche Ausführungsform gezeigt, die als Brustgurt oder als Bauchgurt ausgebildet ist. Dabei kann der Rückenkörper RK senkrecht zur Längsachse LA großflächiger gestaltet sein, um eine zuverlässige Hemmung der Rückenposition beim Träger TR zu bewirken.

Din in Fig. 4 und Fig. 5 gezeigten Ausführungsformen können als einteilige, durchgängig flexible Bandage aus einem Strickmaterial gebildet sein, wobei wiederum eine innenliegende Hakenseite des Klettverschluss vorgesehen ist, so dass keine Abdeckung notwendig ist, um vor versehentlichem Kletten an anderen Materialien zu schützen. Ein breiter Klettbereich ermöglicht ein einfaches Anlegen der Bandage und einen sicheren Halt während des Tragens. Der Hemmkörper HK kann um bis zu 15 cm höher als Bandage ausgeführt sein, um das Ziel der Seitenlage zu erreichen. Die Breite des Hemmkörpers kann auch bis zu Rückenbreite des Trägers gewählt sein.

Die vorstehend und die in den Ansprüchen angegebenen sowie die den Abbildungen entnehmbaren Merkmale sind sowohl einzeln als auch in verschiedener Kombination vorteilhaft realisierbar. Die Erfindung ist nicht auf die beschriebenen Ausführungsbeispiele beschränkt, sondern im Rahmen fachmännischen Könnens in mancherlei Weise abwandelbar.

## Patentansprüche

1. Vorrichtung zur Verhinderung von Schnarchen, die einen Grundkörper (GK) aufweist, der um eine Rückenseite (RS) und eine Vorderseite (VS) eines Trägers (TR) anbringbar ist, wobei der Grundkörper (GK) auf der Rückenseite (RS) einen Rückenkörper (RK) aufweist, in den ein vorstehender Hemmkörper (HK) mit im wesentlichen länglicher Form entlang einer Längsachse (LA) eingebracht ist, wobei der Rückenkörper (RK) so angeordnet ist, dass die Längsachse (LA) im wesentlichen senkrecht zu einer Längsachse einer Wirbelsäule (WS) des Trägers (TR) liegt.

2. Vorrichtung nach Anspruch 1, bei der der Rückenkörper (RK) entlang der Längsachse (LA) eine Länge aufweist, die im Wesentlichen der Breite der Rückenseite (RS) entspricht.

3. Vorrichtung nach Anspruch 1, bei der die Länge des Rückenkörpers (RK) entlang der Längsachse (LA) kleiner als die Breite der Rückenseite gewählt ist.

4. Vorrichtung nach einem der Ansprüche 1 bis 3, bei der der Rückenkörper (RK) entlang der Längsachse (LA) mit einem rechteckigen Querschnitt ausgeführt wird.

5. Vorrichtung nach einem der Ansprüche 1 bis 4, bei der der Rückenkörper (RK) mit einem Verschluss versehen ist, so dass ein auswechselbares Polster in dem Rückenkörper als Hemmkörper (HK) angeordnet werden kann.

6. Vorrichtung nach einem der Ansprüche 1 bis 5, bei der der Grundkörper (GK) als Weste ausgeführt ist.

7. Vorrichtung nach Anspruch 6, bei der die Weste mit einem Klettverschluss an der Vorderseite oder mit einem Reißverschluss versehen ist.

8. Vorrichtung nach einem der Ansprüche 1 bis 5, bei der der Grundkörper (GK) als Bauchgurt ausgeführt ist.

9. Vorrichtung nach einem der Ansprüche 1 bis 5, bei der der Grundkörper (GK) als Brustgurt ausgeführt ist.

10. Vorrichtung nach einem der Ansprüche 1 bis 9, bei der der Hemmkörper (HK) in Richtung der Rückenseite des Trägers profiliert ausgeführt ist.

11. Vorrichtung nach einem der Ansprüche 1 bis 10, bei der der Hemmkörper (HK) als Schaumstoffblock in den Rückenkörper eingebracht ist.

12. Vorrichtung nach einem der Ansprüche 1 bis 10, bei der der Hemmkörper (HK) als aufblasbarer oder fixierter Hohlkörper in den Rückenkörper eingebracht ist.
